(19) Europäisches Patentamt
European Patent Office
Office européen des brevets

(11) Veröffentlichungsnummer: **0 318 845 B1**

(12) # EUROPÄISCHE PATENTSCHRIFT

(45) Veröffentlichungstag der Patentschrift: **16.09.92**

(51) Int. Cl.5: **C07D 213/50**, C07D 213/80

(21) Anmeldenummer: **88119631.5**

(22) Anmeldetag: **25.11.88**

(54) **Verfahren zur Herstellung von substituierten Pyridinen.**

(30) Priorität: **04.12.87 DE 3741160**

(43) Veröffentlichungstag der Anmeldung:
**07.06.89 Patentblatt 89/23**

(45) Bekanntmachung des Hinweises auf die Patenterteilung:
**16.09.92 Patentblatt 92/38**

(84) Benannte Vertragsstaaten:
**BE CH DE FR GB LI NL**

(56) Entgegenhaltungen:
**EP-A- 0 131 887**
**EP-A- 0 232 182**
**US-A- 4 220 783**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Goetz, Norbert, Dr.**
**Schoefferstrasse 25**
**W-6520 Worms 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Verfahren zur Herstellung von substituierten Pyridinen durch katalytische Umsetzung von Gemischen aus Acrolein mit Alkanalen bzw. Ketonen und Ammoniak in Gegenwart von Zeolithkatalysatoren.

Eine Übersicht über die verschiedenen Herstellverfahren von Pyridin und substituierten Pyridinen sowie die Verwendung derartiger Pyridinbasen findet sich in Chem. Techn. 19 (9), S. 528-537 (1967).

Es ist bekannt, daß bei der Umsetzung von Acrolein mit Ammoniak in der Gasphase in Gegenwart von Katalysatoren 3-Methylpyridin entsteht. Als Katalysatoren dienen insbesondere bei Temperaturen von 550 bis 1200 °C mit Sauerstoff vorbehandelte Verbindungen aus den Elementen Al, F und O, die zusätzlich mindestens ein Element der zweiten, dritten oder vierten Gruppe des Periodensystems (DE-OS 21 51 417) oder mindestens zwei Elemente der zweiten, vierten, fünften oder sechsten Gruppe des Periodensystems (DE-OS 22 24 160) oder mindestens ein Element der zweiten Hauptgruppe des Periodensystems (DE-OS 22 39 801) enthalten. Es ist auch bekannt, bei Ausführung der Umsetzung im Wirbelbett das Acrolein getrennt vom Ammoniak in die Wirbelschicht einzuspeisen (DE-OS 24 49 340). Nachteilig ist bei diesen Verfahren, daß neben 3-Methylpyridin in erheblichem Umfang Pyridin entsteht und die Ausbeute an 3-Methylpyridin unter 30% liegt.

Es ist außerdem bekannt, zur Herstellung von 3-Methylpyridin Gemische von Acrolein und Propionaldehyd mit Ammoniak umzusetzen. Als Katalysatoren werden Aluminiumoxid, Siliciumoxid oder Siliciumoxid in Mischung mit 5 bis 50 % Aluminiumoxid, gegebenenfalls mit Zusätzen von Oxiden weiterer Elemente verwendet (FR-PS 1 273 826). Bei diesem Verfahren beträgt die Ausbeute an 3-Methylpyridin höchstens 53 %.

In DE-PS 2 703 070 wird durch Einsatz von hochdispersen Aluminiumsilikaten mit einer BET-Oberfläche von 200 bis 800 $m^2/g$ die Ausbeute an 3-Methylpyridin im Vergleich zu den voranbeschriebenen Verfahren auf bis zu 61 % gesteigert.

Aus DE-PS 2 819 196 ist die Herstellung von aromatisch und heteroaromatisch substituierten Pyridinen bekannt. An bei 550 bis 1200 °C vorbehandelten Verbindungen aus den Elementen Al, F und O, die zusätzlich ein Element der 2., 3. oder 4. Gruppe des Periodensystems enthalten, werden Ausbeuten bis 65 % Phenylpyridin erhalten.

Auch Lanthan dotierte zeolithische Molekularsiebe (DE-PS 2 023 158) sind nur mäßig wirksame Katalysatoren und ergeben daher nur geringe Ausbeuten. Nachteilig ist hierbei auch, daß die Reaktion lediglich auf den Einsatz von Acrolein beschränkt ist, in Gegenwart von Sauerstoff - womit Sicherheitsrisiken verbunden sind - ausgeführt wird und ein Gemisch aus Pyridin (43 mol-%) und 3-Methylpyridin (22 mol-%) erhalten wird.

In US-PS 4 220 783 wird ein Verfahren zur Herstellung von Pyridin und Picolin an Alumosilikatzeolith ZSM 5 aus $C_2$- bis $C_4$-Aldehyden oder $C_3$- bis $C_5$-Ketonen mit $NH_3$ beschrieben. Die Reaktion erfolgt in Gegenwart von Methanol oder Wasser. In Gegenwart von Formaldehyd wird mehr Pyridin gebildet. Der Katalysator desaktiviert sehr schnell; nach 0,7 h werden 93 % Umsatz und nach 3 h nur noch 78 % Umsatz gemessen. Auch die Ausbeute mit 7,7 % Pyridin und 59,6 % Picolin läßt zu wünschen übrig. In Gegenwart der anderen genannten Katalysatoren werden noch schlechtere Ausbeuten erhalten; es treten mehr Kohlenwasserstoffe und hochsiedende Produkte auf.

Aus EP 131 887 ist bekannt, daß acide Alumosilikatzeolithe vom Pentasiltyp mit einem Constraint Index von 1 bis 12 im Wirbelbett für die Herstellung von Alkylpyridinen bessere Ergebnisse liefern als im Festbett (vgl. US-PS 4 220 783). Bei der Umsetzung von Acetaldehyd mit Formaldehyd beträgt die höchste Gesamtausbeute der Summe der erhaltenen Pyridine 89,8 %, wobei das Pyridin/ß-Picolin-Verhältnis gleich 2:1 ist. Der Nachteil dieses Verfahrens ist, daß der Katalysator bereits nach kurzer Zeit regeneriert werden muß und das Produktspektrum selbst bei kurzen Laufzeiten nicht konstant gehalten werden kann.

Es bestand die technische Aufgabe, mit funktionellen Gruppen substituierte Pyridine aus leicht zugänglichen Ausgangsstoffen selektiv zu synthetisieren. Weiterhin sollen sich die hierfür eingesetzten Katalysatoren durch hohe Aktivität und lange Standzeit auszeichnen.

Es wurde gefunden, daß man die Nachteile der bekannten Verfahren vermeidet und substituierte Pyridine der Formel (I)

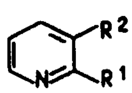

(I)

durch Umsetzung von Acrolein mit Alkanalen oder deren Acetale der Formel (II) oder Ketonen der Formel

(III)

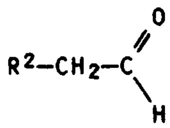 (II) und 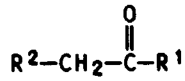 (III)

in der $R^1$ Wasserstoff, Alkyl- mit 1 bis 12 Kohlenstoffatomen, Cycloalkyl-, Aryl- oder Aralkylreste und $R^2$ Acyl- oder Carbonylalkoxi-Reste bedeuten, und Ammoniak in Gegenwart von Katalysatoren in guten Ausbeuten aus leicht zugänglichen Ausgangsstoffen erhält, dadurch gekennzeichnet, daß man die Umsetzung in Gegenwart von Zeolithen bei Temperaturen von 20 bis 500 °C und Drücken von 0,1 bis 100 bar durchführt.

Die Reaktion wird vorzugsweise in der Gasphase bei einer Temperatur von 100 bis 500 °C ausgeführt.

Es war überraschend, daß die Synthese von Acrolein in Gegenwart von Ammoniak mit bifunktionellen Verbindungen selektiv verläuft, da man hierbei mit intermolekularen Reaktionen bzw. Folgereaktionen der Edukte und Produkte hatte rechnen müssen.

Ausgangsstoffe sind Acrolein und Alkanale bzw. deren Acetale und Ketone der Formel (II) und (III). Geeignete Verbindungen sind z.B. Acetylaceton, 1,1-Dimethoxibutan-3-on, Acetylacetaldehyd, Acetessigsäuremethylester, Acetessigsäurepropylester, $\alpha,\beta,\gamma$-Ketocarbonsäureester und Propionylaceton.

Die erfindungsgemäße Umsetzung läßt sich z.B. für den Fall von 2-Methyl-, 3-Acetylpyridin durch folgende Formelgleichung wiedergeben:

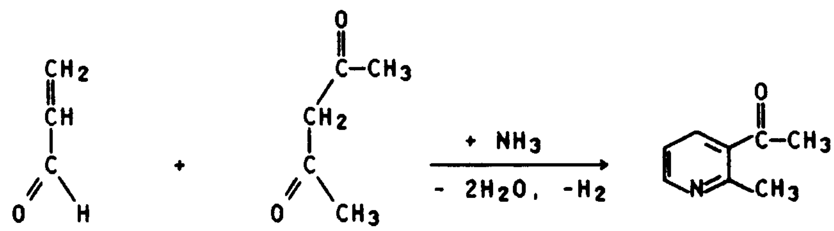


Als Katalysatoren für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie der techn. Chemie, 4. Auflage, Band 24, Seite 575 (1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites" Band 5 aus "Studies in Surface Science and Catalysis" ed. B. Imelik et. al. Elsevier Scientific Publishing Comp. 1980, S. 203, "Crystal Structures of Ultra-stable Faujasites" Advances in Chemistry Series Nr. 101, American Chemical Society Washington, DC, S. 226ff (1971) und US-PS 4 512 961.

Besonders vorteilhaft verwendet man die Zeolithe vom Pentasiltyp. Diese haben als Grundbaustein

einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und enen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithode oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps. Der Aluminosilikatzeolith kann aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder ohne Alkali- oder Erdalkalizusatz bei 100 bis 220 °C unter autogenem Druck hergestellt werden. Auch die isotaktischen Zeolithe nach DE-OS 3 006 471 sind geeignet. Die erhaltenen Aluminosilikatzeolithe enthalten je nach Wahl der Ausgangsstoffmengen ein $SiO_2/Al_2O_3$-Verhältnis von 10 bis 40.000. Die Aluminosilikatzeolithe können in etherischem Medium wie Diethylenglykoldimethylether, in alkoholischem Medium wie Methanol bzw. 1,4-Butandiol oder in Wasser synthetisiert werden.

Der Borosilikatzeolith kann bei 90 bis 200 °C unter autogenem Druck synthetisiert werden, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt. Auch die oben erwähnten isotaktischen Zeolithe nach DE-OS 3 006 471 und EP-PS 46 504 sind geeignet. Solche Borosilikatzeolithe können auch hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendbaren siliciumreichen Zeolithen ($SiO_2/Al_2O_3 \geqq 10$) gehören auch die sog. ZSM-Typen wie sie z.B. in US-PS 3 702 886 u.a. beschrieben sind; Ferrierit, ein kristalliner Zeolith (EP-PS 12 473); NU-1, ein kristalliner Zeolith (US-PS 4 060 590) sowie Silicalit®, ein Molekularsieb, ein sog. Silica Polymorph (US-PS 4 061 724).

Die so hergestellten Alumino-, Boro- und Eisensilikatzeolithe können nach ihrer Isolierung, Trocknung bei 100 bis 160 °C, insbesondere 110 °C und Calcinierung bei 450 bis 550 °C, insbesondere 500 °C, mit einem Bindemittel im Verhältnis 90:10 bis 40:60 Gew.% zu Strängen oder Tabletten verformt werden. Als Bindemittel eignen sich verschiedene Aluminiumoxide, bevorzugt Boehmit, amorphe Aluminosilikate mit einem $SiO_2/Al_2O_3$-Verhältnis von 25:75 bis 90:5, insbesondere 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 g getrocknet und bei 500 °C/16 h calciniert.

Man erhält auch vorteilhafte Katalysatoren, wenn die isolierten Alumino-bzw. Borosilikatzeolithe direkt nach der Trocknung verformt werden und erst nach der Verformung einer Calcinierung unterworfen werden. Die Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden können.

Liegen die Zeolithe aufgrund der Art der Herstellung nicht in der katalytisch aktiven, aciden H-Form vor, sondern z.B. in der Na-form, dann kann diese durch Ionenaustausch mit Ammoniumionen und anschließende Calcinierung oder durch Behandlung mit Säuren vollkommen oder partiell in die gewünschte H-Form überführt werden.

Wenn an den zeolithischen Katalysatoren während der Reaktion eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, insbesondere bei 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Ir, Ni, Pd, Pt, Übergangsmetalle

der 1. und 2. Nebengruppe we Cu, Ag, Zn, Seltene Erdmetalle wie La, Ce, Pr, Nd, Er, Yb und U eingesetzt.

Zweckmäßigerweise führt man die Dotierung so durch, daß man verformte Zeolithe in einem Steigrohr vorlegt und bei 20 bis 100 °C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform der Zeolithe vorgenommen werden. Eine weitere Möglichkeit der Metallaufbringung auf die Zeolithe ist gegeben, indem man das zeolithische Matrial z.B. mit einem Halogenid, einem Nitrat oder einem Oxid der oben beschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser gelöst. Mit dieser Lösung werden die verformten oder unverformten Zeolithe eine gewisse Zeit, z.B. 30 Minuten, getränkt. Über stehende Lösung wird am Rotationsverdampfer von Wasser befreit. Danach werden die getränkten Zeolithe bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin die reinen pulverförmigen Zeolithe bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch an den in der H-, Ammonium- oder Alkali-Form vorliegenden Zeolithen kann so vorgenommen werden, daß man die Zeolithe in Strängen oder Pellets in einer Kolonne vorgelegt und darüber eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft so vor, daß man die Zeolithe in Pulverform mit 1 n Phosphorsäure 1 Stunde bei 80 °C behandelt. Nach der Behandlung wird mit Wasser gewaschen, bei 100 °C/16 Stunden getrocknet und bei 500 °C/20 Stunden calciniert. Nach einer anderen Arbeitsweise behandelt man die Zeolithe vor oder nach ihrer Verformung mit Bindemitten, z.B. 1 bis 3 Stunden bei Temperaturen von 60 bis 80 °C mit einer 3 bis 25 gew.%igen, insbesondere 12 bis 20 gew.%igen wäßrigen Salzsäure. Anschließend werden die so behandelten Zeolithe mit Wasser gewaschen, getrocknet und bei 400 °C bis 500 °C calciniert.

Eine besondere Ausführungsform der Säurebehandlung besteht darin, daß man das zeolithische Material vor seiner Verformung bei erhöhter Temperatur mit 0,001 n bis 2 n, insbesondere 0,05 n bis 0,5 n Flußsäure durch Erhitzen unter Rückfluß über einen Zeitraum von 0,5 bis 5, insbesondere 1 bis 3 Stunden behandelt. Nach Isolierung durch Abfiltrieren und Auswaschen wird das zeolithische Material zweckmäßig bei Temperaturen von 450 bis 600 °C calciniert. Nach einer anderen bevorzugten Ausführungsform für die Säurebehandlung wird das zeolithische Material nach einer Verformung mit Bindemittel bei erhöhter Temperatur, zweckmäßig bei Temperaturen von 50 bis 90 °C, insbesondere 60 bis 80 °C über einen Zeitraum von 0,5 bis 5 h mit 12 bis 20 gew.%iger Salzsäure, behandelt. Anschließend wird das zeolithische Material ausgewaschen und bei 100 bis 160 °C getrocknet und bei 450 bis 600 °C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 100 °C getrocknet und bei 500 °C calciniert.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Bei dem erfindungsgemäßen Verfahren werden zweckmäßig folgende Reaktionsbedingungen gewählt: Das Molverhältnis von Acrolein:Alkanal bzw. Keton der Formel (II) bzw. (III):$NH_3$ soll 1:1 bis 2:1 bis 15 molar, bevorzugt 1:1:3 bis 6 molar sein. Die Umsetzung wird zweckmäßig in der Gasphase bei Temperaturen von 100 bis 500 °C durchgeführt. Vorteilhaft hält man eine Temperatur von 200 bis 450 °C, insbesondere 250 bis 400 °C ein. In der Regel führt man die Umsetzung bei einem Druck von 0,1 bis 100 bar,

insbesondere 0,5 bis 10 bar durch.

Beim Umsetzen von Acrolein und Alkanalen der Formel (II) bzw. Ketonen der Formel (III) mit Ammoniak an den oben beschriebenen Katalysatoren in der Gasphase wird vorteilhaft eine Katalysatorbelastung von 0,1 bis 20, insbesondere von 1 bis 10 g Gemisch Acrolein und Alkanal/Keton je g Katalysator und Stunde (WHSV = g Einsetzgemisch/g Katalysator und Stunde) eingehalten.

Die Gasphasenreaktion kann in einem Festbett- oder einem Wirbelbettreaktor ausgeführt werden.

Es ist auch möglich, die Reaktion in der Flüssigphase (Suspension-, Riesel- oder Sumpffahrweise) bei Temperaturen zwischen 20 und 200 °C durchzuführen.

Die Reaktion kann diskontinuierlich, vorzugsweise kontinuierlich unter Normaldruck, Druck oder Unterdruck durchgeführt werden. Schwerflüchtige oder feste Ausgangsstoffe werden in gelöster Form, z.B. in THF-, Toluol- oder Petrolether-Lösung zum Einsatz gebracht. Im allgemeinen ist eine Verdünung des Ausgangsstoffes mit Lösungsmitteln oder mit Inertgasen wie $N_2$, Ar, $H_2O$-Dampf möglich.

Nach der Umsetzung werden die entstandenen Produkte durch übliche Verfahren, z.B. durch Destillation aus dem Reaktionsgemisch isoliert; nicht umgesetzte Ausgangsstoffe werden gegebenenfalls in die Umsetzung zurückgeführt.

Die nach dem erfindungsgemäßen Verfahren hergestellten substituierten Pyridine sind vielseitig verwendbare Zwischenprodukte.

Beispiel 1 bis 8

Die Reaktion in der Gasphase wird unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch nach bekannter Methode.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit Boehmit im Gewichts-verhältnis 60:40 2-mm-Stränge hergestellt, die bei 110 °C/16 h getrocknet und bei 500 °C/24 h calciniert werden.

Katalysator B

Ein Aluminosilikatzeolith vom Pentasil-Typ wird unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Aluminosilikatzeolith enthält 91,6 Gew.% $SiO_2$ und 4,6 Gew.% $Al_2O_3$. Der Katalysator wird durch Verformen mit Boehmit im Gewichtsverhältnis 60:40 zu 2-mm-Strängen erhalten, bei 110 °C/16 h getrocknet und bei 500 °C/24 h calciniert.

Katalysator C

100 g des als Katalysator A erhaltenen Borosilikatzeolithen werden mit 280 ml einer 0,1 n HF bei 90 °C 2 h lang behandelt und nach Abfiltrieren bei 160 °C getrocknet. Dieses Produkt wird mit amorphem Aluminosilikat (25 Gew.% $Al_2O_3$ und 75 Gew.% $SiO_2$) im Gewichtsverhältnis 60:40 zu 2-mm-Strängen verformt, bei 110 °C/16 h getrocknet und bei 500 °C/16 h calciniert.

Katalysator D

Handelsüblicher NaY-Zeolith wird mit Boehmit im Gewichtsverhältnis 60:40 zu 2-mm-Strängen verformt, bei 100 °C/16 h getrocknet und bei 540 °C/24 h calciniert. Diese Stränge werden mit einer 20 %igen wäßrigen $La(NO_3)_2$-Lösung bei 80 °C/2 h ionenausgetauscht. Nach Trocknung bei 100 °C und Calcination

bei 500 °C soll der La-Gehalt 7,1 Gew.% und der Na-Gehalt 1,1 Gew.% betragen. Der Ionenaustausch kann nach Zwischencalcination wiederholt werden bis obige La- bzw. Na-Gehalte eingestellt sind.

Katalysator E

Katalysator A wird mit wäßriger $Ce(NO_3)_3$-Lösung imprägniert, bei 130 °C/2 h getrocknet und bei 540 °C/2 h calciniert. Der Ce-Gehalt beträgt 2,5 Gew.%.

Versuchsergebnisse und Reaktionsbedingungen sind in der folgenden Tabelle zusammengestellt.

Tabelle 1

| Acrolein (I) + Ausgangsstoff (II) + $NH_3$ → (III) | | | | | | |
|---|---|---|---|---|---|---|
| Beispiel | 1 | 2 | 3 | 4 | 5 | 6 |
| Ausgangsstoff II | Acetylaceton | | | | | |
| Katalysator | A | B | C | D | D | E |
| Temperatur(°C) | 350 | 350 | 350 | 350 | 300 | 350 |
| WHSV $h^{-1}$ | 3,0 | 3,0 | 3,0 | 3,0 | 3,0 | 3,0 |
| I:II:$NH_3$ (molar) | 1:1:3 | 1:1:3 | 1:1:3 | 1:1:3 | 1:1:3 | 1:1:3 |
| [1] Umsatz (%) | 100 | 100 | 100 | 100 | 100 | 100 |
| [2] Selektivität (%) | 79,0 | 58,2 | 66,5 | 64,9 | 72,7 | 64,0 |

[1] Umsatz sowohl an Acrolein als auch an Ausgangsstoff II

[2] Gemessen als 2-Methyl-3-acetylpyridin (III) - Nebenprodukte sind: Pyridin, 2- und 3-Methylpyridin, Acetamid, Acetonitril, Dimethylpyridin, 2-Amino-2-penten-4-on

Beispiel 7

Acrolein wird in der beschriebenen Apparatur mit 1,1-Dimethoxy-butan-3-on und Ammoniak im molaren Verhältnis 1:1:6 bis 350 °C und WHSV-1,5 $h^{-1}$ an Katalysator C umgesetzt. Der Umsatz beträgt an Acrolein jeweils 100 %. Es werden vornehmlich Methanol und 3-Acetylpyridin gebildet, als Nebenprodukte entstehen Aceton, Acetonnitril, Pyridin, Methylpyridine. Die Selektivität an 3-Acetylpyridin beträgt 52,4 %. Bei einer WHSV-3 $h^{-1}$ liegt diese Selektivität bei 56,9 %.

Beispiel 8

Acrolein wird in der beschriebenen Apparatur mit Acetylessigsäure-methylester und Ammoniak im molaren Verhältnis 1:1:3 bei 350 °C und WHSV-2,5 $h^{-1}$ an Katalysator C umgesetzt. Der Umsatz beträgt an beiden Ausgangsstoffen 100 %. Die Selektivität für 2-Methyl-3-nicotinsäuremethylesterpyridin beträgt 45,7 %. Nebenprodukte sind z.B. 2-Methyl-3-cyanopyridin, 2-Methyl-3-nictoinsäureamid, 2- und 3-Methylpyridin.

## Patentansprüche

**1.** Verfahren zur Herstellung von substituierten Pyridinen der Formel (I)

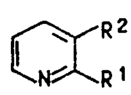

(I)

durch Umsetzung von Acrolein mit Alkanalen oder deren Acetale der Formel (II) oder Ketonen der Formel (III)

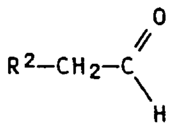 (II) , 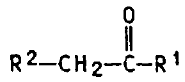 (III)

in der $R^1$ Wasserstoff, Alkyl- mit 1 bis 12 Kohlenstoffatomen, Cycloalkyl-, Aryl- oder Aralkylreste und $R^2$ Acyl- oder Carbonylalkoxi-Reste bedeuten, und Ammoniak in Gegenwart von Katalysatoren, dadurch gekennzeichnet, daß man die Umsetzung in Gegenwart von Zeolithen bei Temperaturen von 20 bis 500 ° C und Drücken von 0,1 bis 100 bar durchführt.

**2.** Verfahren nach Anspruch 1, dadurch gekennzeichnet, daß man Acrolein mit Acetylaceton, 1,1-Dimethoxi-butan-3-on (Ketobutyraldehyddimethylacetal) oder Acetessigsäuremethylester einsetzt.

**3.** Verfahren nach Anspruch 1, dadurch gekennzeichnet, daß man als Katalysatoren Zeolithe des Pentasiltyps verwendet.

**4.** Verfahren nach Anspruch 1, dadurch gekennzeichnet, daß man als Katalysatoren Bor-, Eisen- und/oder Aluminiumsilikatzeolithe des Pentasiltyps verwendet.

**5.** Verfahren nach Anspruch 1, dadurch gekennzeichnet, daß man als Katalysatoren Aluminiumsilikatzeolithe des Faujasittyps verwendet.

**6.** Verfahren nach Anspruch 1, dadurch gekennzeichnet. daß man als Katalysatoren mit Alkali-, Erdalkali-, Übergangsmetallen oder mit Seltenen Erden dotierte Zeolithe verwendet.

**7.** Verfahren nach Anspruch 1, dadurch gekennzeichnet, daß man die Umsetzung in der Gasphase durchführt.

## Claims

**1.** A process for the preparation of a substituted pyridine of the formula (I)

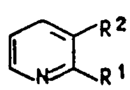 (I)

by reacting acrolein with an alkanal or an acetal thereof of the formula (II) or a ketone thereof of the formula (III)

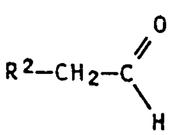 (II) 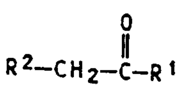 (III)

where $R^1$ is hydrogen, alkyl of 1 to 12 carbon atoms, cycloalkyl, aryl or aralkyl and $R^2$ is acyl or carbonylalkoxy, and ammonia in the presence of a catalyst, wherein the reaction is carried out in the presence of a zeolite at from 20 to 500 ° C and from 0.1 to 100 bar.

**2.** A process as claimed in claim 1, wherein acrolein is used with acetylacetone, 1,1-dimethoxybutan-3-one (ketobutyraldehyde dimethyl acetal) or methyl acetoacetate.

**3.** A process as claimed in claim 1, wherein the catalyst used is a zeolite of the pentasil type.

**4.** A process as claimed in claim 1, wherein the catalyst used is a boron silicate, iron silicate or aluminum silicate zeolite of the pentasil type.

5. A process as claimed in claim 1, wherein the catalyst used is an aluminum silicate zeolite of the faujasite type.

6. A process as claimed in claim 1, wherein the catalyst used is a zeolite doped with alkali metals, alkaline earth metals, transition metals or rare earth metals.

7. A process as claimed in claim 1, wherein the reaction is carried out in the gas phase.

**Revendications**

1. Procédé de préparation de pyridines substituées répondant à la formule (I)

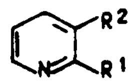

(I)

par la réaction de l'acroléine avec des alcanals, ou leurs acétals, de la formule (II), ou des cétones de la formule (III)

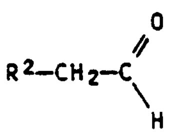

(II) ,

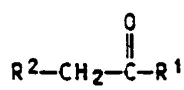

(III)

formules dans lesquelles $R^1$ représente un atome d'hydrogène, des radicaux alkyle comportant de 1 à 12 atomes de carbone, cycloalkyle, aryle ou aralkyle et $R^2$ représente des radicaux acyle ou carbonylalcoxy, et l'ammoniac, en présence de catalyseurs, caractérisé en ce que l'on entreprend la réaction à des températures de 20 à 500 °C et sous des pressions de 0,1 à 100 bars, en présence de zéolites.

2. Procédé suivant la revendication 1, caractérisé en ce que l'on utilise l'acroléine avec l'acétylacétone, la 1,1-diméthoxy-butane-3-one (cétobutyraldéhydediméthylacétal), ou l'acétoacétate de méthyle.

3. Procédé suivant la revendication 1, caractérisé en ce que l'on utilise des zéolites du type pentasyle à titre de catalyseurs.

4. Procédé suivant la revendication 1, caractérisé en ce que l'on utilise des zéolites du type silicate de bore, de fer et/ou d'aluminium du type pentasyle à titre de catalyseurs.

5. Procédé suivant la revendication 1, caractérisé en ce que l'on utilise des zéolites de silicate d'aluminium du type faujasite à titre de catalyseurs.

6. Procédé suivant la revendication 1, caractérisé en ce que l'on utilise des métaux alcalins, des métaux alcalino-terreux, des métaux de transition, ou des zéolites dopées à l'aide de terres rares, à titre de catalyseurs.

7. Procédé suivant la revendication 6, caractérisé en ce que l'on entreprend la réaction en phase gazeuse.